(19) Europäisches Patentamt
European Patent Office
Office européen des brevets

(11) **EP 1 760 119 A1**

(12) **EUROPEAN PATENT APPLICATION**
published in accordance with Art. 158(3) EPC

(43) Date of publication:
**07.03.2007 Bulletin 2007/10**

(21) Application number: **05728926.6**

(22) Date of filing: **30.03.2005**

(51) Int Cl.:
*C09C 3/10* *(2006.01)*    *C09C 1/04* *(2006.01)*
*C09C 1/36* *(2006.01)*

(86) International application number:
**PCT/JP2005/006722**

(87) International publication number:
**WO 2005/100487 (27.10.2005 Gazette 2005/43)**

(84) Designated Contracting States:
**DE FR GB**

(30) Priority: **31.03.2004 JP 2004102711**

(71) Applicant: **KAO CORPORATION**
**Tokyo 103-8210 (JP)**

(72) Inventors:
• **KUWAHARA, Kazuo c/o KaoCorporation**
**Wakayama-shi (JP)**

• **OHNO, Kohji**
**Souraku-gun Kyogo (JP)**
• **FUKUDA, Takeshi**
**Kyoto (JP)**

(74) Representative: **HOFFMANN EITLE**
**Patent- und Rechtsanwälte**
**Arabellastrasse 4**
**81925 München (DE)**

(54) **METAL OXIDE COMPOSITE OXIDE AND COSMETIC INCLUDING THE SAME**

(57)    The present invention relates to metal oxide complex powder obtained by polymerizing a polymerizable and unsaturated group-containing monomer via a polymerization initiation group introduced onto the surface of at least one metal oxide selected from zinc oxide, titanium oxide and cerium oxide and cosmetics containing the metal oxide complex power.

**Description**

Field of the invention

The present invention relates to metal oxide complex powder and cosmetics containing the same.

Background of the invention

Metal oxides have specific coloring power and are thus incorporated as pigments into cosmetics, and particularly zinc oxide and titanium oxide are incorporated into cosmetics for purposes such as ultraviolet-shielding properties and antibacterial properties. The surfaces of these metal oxides are hydrophilic so that the metal oxides cannot be incorporated as such into oily cosmetics. As such, the metal oxides are poor in the touch and inferior in feel for use. For incorporation into cosmetics, therefore, the metal oxides are often surface-treated with methyl hydrogen siloxane·organosiloxane or with organic compounds having perfluoroalkyl groups, to improve hydrophobic properties and feel (for example JP-A 11-80588). To solve these problems, it is disclosed that these metal oxides are dispersed in monomers and then subjected to suspension polymerization/emulsion polymerization in water to form metal oxide complex powder (JP-B 3205249).

Summary of the invention

The present invention relates to metal oxide complex powder obtained by polymerizing a polymerizable and unsaturated group-containing monomer via a polymerization initiation group introduced onto the surface of at least one metal oxide selected from zinc oxide, titanium oxide and cerium oxide and cosmetics containing the metal oxide complex power. The present invention also provides use of the metal oxide complex powder for cosmetics.

Detailed description of the invention

In the surface treatment of a metal oxide in one prior art mentioned above, the metal oxide is surface-treated in an aggregated state, and thus untreated portions of the metal oxide are exposed outside by mechanical force upon blending, to bring about problems such as deterioration in feel, reduction in compound stability, etc. In forming a complex of a metal oxide and resin powder in the other prior art, there is a problem that by formation of a complex with a polymer by suspension polymerization/emulsion polymerization, the particle diameter of the resulting complex particle is increased even if the particle diameter of the metal oxide is reduced, so the complex may remain white at use and the stability of the resulting compound may be lowered. There is also a problem that when a metal oxide is incorporated into a polycarboxylic acid-based aqueous thickening agent, the viscosity is reduced with time due to interaction of the metal oxide such as zinc oxide with the thickening agent.

Accordingly, the present invention provides metal oxide complex powder by which the particle diameter can be reduced, the exposure of the metal oxide surface by mechanical strength upon blending can be suppressed, and the metal oxide does not reduce the viscosity even when used in combination with a polycarboxylic acid-based thickening agent, as well as cosmetics excellent in feel and compound stability containing the same.

According to the present invention, there can be obtained metal oxide complex powder coated with a polymer while a metal oxide is hardly aggregated, to suppress the exposure of the metal oxide by mechanical force upon blending, and there can be provided cosmetics excellent in feel and compound stability. Even if the metal oxide complex powder is incorporated into cosmetics containing a polycarboxylic acid-based aqueous thickening agent, a reduction in viscosity with time can be suppressed, and thus there can be provided cosmetics excellent in viscosity stability.

[Metal oxide complex powder and a process for producing the same]

The metal oxide complex powder of the present invention is obtained by polymerizing polymerizable unsaturated group-containing monomers via a polymerization initiation group introduced onto the surface of a metal oxide (hereinafter, this operation refers sometimes to surface grafting, and a polymer chain formed from the surface of the metal oxide by this operation refers sometimes to graft chain.). In the metal oxide complex powder of the present invention, the metal oxide is present inside of the complex powder.

The metal oxide used in the present invention includes at least one member selected from zinc oxide, titanium oxide and cerium oxide having a high ultraviolet-protecting ability, among which zinc oxide is preferable. The metal oxide can be used regardless of its shape, and a spherical, plate-shaped or amorphous metal oxide can be used. Depending on applications, metal oxides different in shape can be simultaneously used.

The average particle diameter of the metal oxide, in terms of primary particle diameter, is preferably 1 $\mu$m or less, more preferably 0.005 to 1 $\mu$m, still more preferably 0.01 to 0.8 $\mu$m, from the viewpoint of improving the ultraviolet-protecting ability and the transparency of the complex powder and a coating of cosmetics.

The primary particle diameter of the metal oxide is the average of major axes measured at 10 arbitrarily extracted points on a metal oxide particle image obtained from a transmission electron microphotograph.

The method of introducing a polymerization initiation group onto the surface of a metal oxide includes a method of reacting a metal oxide with a compound having a functional group serving as a polymerization initiation group and further having a functional group reacting with the surface of the metal oxide (hereinafter referred to as polymerization initiation compound).

Generally the polymerization initiation compound includes compounds represented by the formula:

$$R^1\text{-}A\text{-}B\text{-}B^2$$

wherein $R^1$ represents a group which is hydrolyzed to form HO- (hydroxyl group) or the like, $R^2$ represents a polymerization initiation group, and A and B each represent an organic group.

Typical examples of the $R^1$ group include an alkoxysilyl group, an alkoxytitanium group, etc. The $R^2$ group includes an azo group, a peroxide group, a persulfate group, a halogenated alkyl group, a halogenated sulfonyl group, etc. When atomic transfer radical polymerization (ATRP) is carried out, the $R^2$ group preferably has a halogenated alkyl group or a halogenated sulfonyl group.

Specific examples of the polymerization initiation compound include 6-(2-bromoisobutyric acid) hexyltrialkoxysilane, 6-(2-bromoisobutyric acid) hexylalkyldialkoxysilane, 6-(2-bromoisobutyric acid) hexyldialkyl monoalkoxysilane, 6-(2-bromoisobutyric acid) hexyltrichlorosilane, 2-(4-chlorosulfonylphenyl) ethyltrialkoxysilane, 2-(4-chlorosulfonylphenyl) ethyltrichlorosilane, etc.

For example, when a polymerization initiation group is to be introduced onto the surface of zinc oxide, 6-(2-bromoisobutyric acid) hexyltriethoxysilane, 6-(2-bromoisobutyric acid) hexyltrichlorosilane or the like can be allowed to react with an oxide layer on the surface of zinc oxide to immobilize a halogenated alkyl group as a polymerization initiation group onto the surface of zinc oxide.

The method of polymerizing monomers via a polymerization initiation group introduced onto the surface of zinc oxide is preferably a radical polymerization method. In consideration of the feel and dispersibility of the metal oxide complex powder, surface grafting at high density while regulating the primary structure of formed polymer chains is considered desirable. As the method of introducing polymer chains, a living radical polymerization method wherein the growth of all graft chains can proceed almost equally to relieve steric hindrance among adjacent graft chains is particularly effective. The living radical polymerization method is preferably an atomic transfer radical polymerization method (ATRP) in the presence of a transition metal complex as described in Chemical Review, 2001, Vol. 101, pp. 2921-2990. The transition metal complex used in this case includes a molybdenum complex, chromium complex, rhenium complex, ruthenium complex, iron complex, rhodium complex, nickel complex, palladium complex, copper complex etc. as illustrated in Chemical Review, 2001, vol. 101, pp. 2935-2940. Further, halogenated copper, and an organic compound capable of coordination with copper, can be added respectively to the reaction system, to form a copper complex in the reaction system. The organic compound capable of coordination with copper used in this case includes sparteine and nitrogen oxides illustrated in Chemical Reviews, 2001, vol. 101, p. 2941. For carrying out surface grafting polymerization, a method of allowing a living radical polymerization initiator such as ethyl 2-bromobutyrate to be present in a free form in a polymerization medium is preferably used as a method effective in regulating a graft chain structure.

The monomers used in production of the metal oxide complex powder of the present invention is not particularly limited insofar as they are radical-polymerizable, and the monomers are more preferably those capable of living radical polymerization, particularly preferably those capable of atomic transfer radical polymerization. These monomers include linear or branched alkyl (meth) acrylates such as methyl (meth)acrylate, ethyl (meth) acrylate, butyl (meth) acrylate, isobutyl (meth)acrylate, t-butyl (meth)acrylate, 2-ethylhexyl (meth)acrylate, dodecyl (meth)acrylate, stearyl (meth)acrylate and isostearyl (meth)acrylate, fluorinated alkyl (meth)acrylates such as 2-(octafluoroalkyl)ethyl (meth)acrylate, (meth)acrylates such as 2-hydroxyethyl (meth)acrylate, polyethylene glycol (meth)acrylate and 1-n-propoxyethyl (meth) acrylate, (meth)acrylamides such as N,N-dimethyl (meth)acrylamide, cationic (meth)acrylates such as N,N-dimethylaminoethyl (meth)acrylate hydrochloride, styrene and styrene type monomers such as p-chlorostyrene, and other monomers such as (meth)acrylonitrile.

As used herein, (meth) acryl refers to acryl or methacryl, (meth) acrylate to acrylate or methacrylate, and (meth) acrylonitrile to acrylonitrile or methacrylonitrile.

The particle diameter of the metal oxide complex powder of the present invention is not particularly limited, but from the viewpoint of feel at use, the primary particle diameter is preferably 1 $\mu$m or less. The particle diameter of the powder is a mean particle diameter determined from major axes measured at 10 arbitrarily extracted points on a transmission electron microscope (TEM) photograph stained negative with an aqueous solution of a heavy-metal compound such as uranyl acetate or phosphotungstic acid.

The content of the metal oxide in the metal oxide complex powder of the present invention is preferably 1 to 95 wt % , more preferably 5 to 90 wt%.

The UV shielding ability ΔT of the metal oxide complex powder of the present invention is preferably 35% or more, more preferably 40% or more. ΔT is a value determined by the following method.

ΔT (%) as a scale of UV shielding ability was determined by dispersing the metal oxide complex powder in an amount of 0.01 wt% in terms of metal oxide in silicone oil 6cs (KF96 6cs manufactured by Shin-Etsu Chemical), then placing the resulting dispersion in a quartz cuvette with a light path length of 50 μm, measuring its transmittances at wavelengths of 550 nm and 350 nm in a spectrophotometer (UV-2550 manufactured by Shimadzu Corporation) and determining ΔT (%) as a difference therebetween as the scale of UV shielding ability.

[Cosmetics]

Cosmetics containing the metal oxide complex powder of the present invention are not particularly limited with respect to their effect/product form, and are preferably those capable of exhibiting the UV shielding effect and skin shielding effect. Preferable examples of the cosmetics include make-up cosmetics such as foundation, rouge, eye shadow, mascara, eye liner, lipstick, nail enamel etc., sunscreen cosmetics, base cosmetics, and hair cosmetics such as hair spray and hair mousse. The content of the metal oxide complex powder in the cosmetics of the present invention is preferably 0.1 to 60 wt%, more preferably 1 to 40 wt%.

The cosmetics of the present invention can be compounded with other components used as usual cosmetic materials suitably selected from for example white pigments (titanium oxide etc.), extender pigments (mica, talc, sericite, barium sulfate etc.), coloring pigments (inorganic coloring pigments such as red oxide, yellow iron oxide, black iron oxide etc. and organic coloring pigments such as Yellow 401, Red 226, etc.), pearl pigment, natural minerals, organic powder, oils (vaseline, lanoline, ceresin, olive oil, jojoba oil, castor oil, squwalane, liquid paraffin, ester oil, diglyceride, silicone oil, perfluoropolyether, fluorine-modified silicon oil, etc.), UV-ray protecting agents, gelling agents, skin-forming agents, wax (solid and semisolid oils such as microcrystalline wax, higher fatty acid and higher alcohol), metal soaps, surfactants, moistening agents, preservatives, perfumes, thickening agents, antioxidants, sterilizers, sweat regulators, and other various additives.

The cosmetics of the present invention can be used in product forms such as solid cosmetics, wax cosmetics, emulsions (water-in-oil type, oil-in-water type) cosmetics, liquid cosmetics, gel cosmetics, sprays and foams.

Examples

Synthesis Example 1

(1) Introduction of a polymerization initiation group into the surface of zinc oxide

5 g fine zinc oxide particles (ZnO-350 with an average particle diameter of 26 nm determined from a TEM photograph, Sumitomo Osaka Cement) and 50 g ethanol were added to a 100-mL glass container equipped with a lid, and 3.4 g of 25% ammonia water was added thereto under stirring. The mixture was stirred as such for 1 hour, and then 5.7 g of 6-(2-bromoisobutyric acid) hexyltriethoxysilane was added thereto. After stirring at room temperature for 24 hours, the reaction mixture was centrifuged (10000 rpm, 20 minutes) to separate zinc oxide into which a polymerization initiation group had been introduced. The zinc oxide was washed 3 times by the same operation using ethanol as a dispersing medium, followed by adding 25 g anisole thereto, whereby a slurry of the zinc oxide into which a polymerization initiation group had been introduced was prepared.

(2) Synthesis of zinc oxide complex powder

A stirrer chip and 0.43 g copper (I) bromide were charged into 200 mL eggplant-type flask and then flushed with nitrogen and cooled with dry ice/methanol as a refrigeration medium. After the material was sufficiently cooled, 100 g methyl methacrylate, 0.2 g ethyl 2-bromoisobutyrate, 1.4 g sparteine, 13.5 g of the slurry of zinc oxide into which a polymerization initiation group had been introduced, prepared in (1) above, and 82 g anisole were added thereto and bubbled with nitrogen for 30 minutes under cooling. After nitrogen replacement was finished, the flask was closed with a three-way cock and heated under heating for 4 hours on a silicone bath at 72°C to polymerize the mixture. After polymerization, the reaction mixture was cooled and centrifuged (10000 rpm x 20 minutes) to recover the solid phase which was then washed with tetrahydrofuran/methanol and dried to give zinc oxide complex powder. Direct measurement of the molecular weight of a surface graft chain of this zinc oxide complex powder is difficult, and from the polymerization mechanism, can be estimated to be almost identical with the molecular weight of a free polymer (a polymer formed ethyl 2-bromoisobutyrate) formed in the solution.

The molecular weight (Mn) of the graft polymer of the zinc oxide complex powder was estimated to be 39000 (polystyrene-equivalent molecular weight) (GPC: Tosoh HLC-8220GPC, columns: G4000HXL + G2000HXL, eluent: tetrahydrofuran), and the molecular-weight distribution (Mw/Mn) was 1.2. The degree of conversion was 42% ($^1$H-NMR: calculated from vinyl $CH_2$ of methyl methacrylate and $\alpha$-$CH_3$ of poly (methyl methacrylate) by Varian MERCURY400). The content of zinc oxide in this powder was 26% (ICP emission analysis: the amount of zinc was measured by ICPS-1000 manufactured by Shimadzu Corporation). As a result of observation with TEM (JEM-2000FX, JEOL Ltd.), it was confirmed that zinc oxide was included in the polymer. From this observation, the diameter of this particle was 0.31 $\mu$m.

Synthesis Example 2

Zinc oxide complex powder was obtained by carrying out the same reaction as in Synthesis Example 1 except that in (2) in Synthesis Example 1, the amount of copper(I) bromide was 0.2 g, the amount of methyl methacrylate was 15 g, the amount of ethyl 2-bromoisobutyrate was 0.14 g, the amount of sparteine was 0.7 g, the amount of the slurry of zinc oxide into which a polymerization initiation group had been introduced was 14 g, and the reaction time was changed to 24 hours. The molecular weight (Mn) of the graft polymer was 14200, the molecular-weight distribution (Mw/Mn) was 1.41, and the degree of conversion was 97% . The content of zinc oxide in this powder was 39%, and it was confirmed that zinc oxide was included in the polymer. The particle diameter was 0.12 $\mu$m.

Synthesis Example 3

In the same manner as in (1) in Synthesis Example 1, zinc oxide was reacted with 6-(2-bromoisobutyric acid) hexyltriethoxysilane, and the reaction mixture was centrifuged, washed, and mixed with 25g of a mixed solvent of methyl ethyl ketone (MEK)/n-propyl alcohol (n-PrOH) = 7/3 (ratio by volume) to prepare slurry of zinc oxide into which a polymerization initiation group had been introduced. Using this slurry, zinc oxide complex powder was obtained by carrying out the reaction in the same manner as in (2) in Synthesis Example 1 except that the amount of copper (I) bromide was 0. 04 g, methyl methacrylate was changed to 15 g of 2-hydroxyethyl methacrylate, sparteine was changed to 0.08 g bipyridine, the amount of the slurry of zinc oxide into which a polymerization initiation group had been introduced was 10 g, and anisole was changed to 17.5 g of a mixed solvent of MEK/n-PrOH = 7/3. The molecular weight (Mn) of the graft polymer was 17000, the molecular-weight distribution (Mw/Mn) was 1.72, and the degree of conversion as determined by GPC was 25%. The content of zinc oxide in this powder was 42%, and it was confirmed that zinc oxide was included in the polymer. The particle diameter was 0.15 $\mu$m.

Synthesis Example 4

Plate-shaped titanium oxide complex powder was obtained in the same manner as in Synthesis Example 1 except that in (1) in Synthesis Example 1, 5 g zinc oxide was changed to 5 g plate-shaped titanium oxide (Luxelene Silk H, Sumitomo Chemicals). The molecular weight (Mn) of the graft polymer was 62000, the molecular-weight distribution (Mw/Mn) was 1.1, the degree of conversion was 40%, and the content of titanium oxide in this powder was 93%. By observation under a scanning electron microscope (SEM), it was confirmed that the shape of the plate-shaped titanium oxide surface had been changed by the polymer.

Comparative Synthesis Example 1

30 g of silicone-treated zinc oxide (MZ-507S) manufactured by Teika was stirred in 70 g methyl methacrylate (SP value, 8.9) for 3 hours with beads mill (the average particle diameter of the zinc oxide as determined by ELS-8000 manufactured by Otsuka Electronics Co., Ltd. was 0.3 $\mu$m). Thereafter, 2.1 g lauroyl peroxide was added thereto. 5 g sodium lauryl sulfate was dissolved in 500 g deionized water, then added to the above-described zinc oxide slurry, and dispersed by means of a milder (the average particle diameter of the emulsion as determined by LA-910 manufactured by Horiba Ltd. was 2.2 $\mu$m). Then, the dispersion was charged into a 2000-mL separable flask, then flushed with nitrogen, and heated to 70˚C under stirring at 200 rpm, then kept at 70˚C for 10 hours, further heated to 80˚C, and subjected to polymerization at 80˚C for 10 hours in a nitrogen atmosphere. After polymerization was finished, the solids were collected by centrifugation, washed with water, lyophilized, and pulverized by a jet mill, to collect 70 g complex polymer particles. The degree of inclusion of zinc oxide in this powder was 40%. By observation under SEM, the average particle diameter was 3 $\mu$m.

The zinc oxide complex powders obtained in Synthesis Examples 1 to 3 and Comparative Synthesis Example 1 were evaluated for their UV shielding ability by the following method. The results are shown in Table 1. The particle diameter and zinc oxide content of each zinc oxide complex powder are also shown in Table 1.

<UV shielding ability>

The zinc oxide complex powder was dispersed in an amount of 0.01 wt% in terms of zinc oxide in silicone oil 6cs (KF96 6cs, manufactured by Shin-Etsu Chemical), placed in a quartz cuvette with a light path length of 50 $\mu$m, and measured for the transmission difference ($\Delta$T) between transmissions at wavelengths of 550 nm and 350 nm in a spectrophotometer (UV-2550 manufactured by Shimadzu Corporation).

Table 1

|  | Synthesis example 1 | Synthesis example 2 | Synthesis example 3 | Comparative synthesis example 1 |
|---|---|---|---|---|
| Particle diameter ($\mu$m) | 0.31 | 0.12 | 0.15 | 3 |
| Zinc oxide content (%) | 26 | 39 | 42 | 40 |
| $\Delta$T (%) | 56 | 54 | 51 | 35 |

Examples 1 to 3, Comparative Examples 1 to 2

The zinc oxide complex powders obtained in Synthesis Examples 1 to 3 and Comparative Synthesis Example 1 and silicone-treated zinc oxide (MZ-507S manufactured by Teika) were used to prepare cosmetics according to the following formulation. The resulting cosmetics were evaluated for compound stability and feel by the following methods. The results are shown in Table 2.

<Cosmetic Formulation>
Zinc oxide complex powder or MZ-507S                                           5 wt%
Ethanol                                                                         15 wt%
Carbopol EDT2020 (polycarboxylic acid-based thickening agent manufactured by Noveon)   0.3 wt%
PEMULEN TR-2 (polycarboxylic acid-based thickening agent manufactured by Noveon)       0.2 wt%
Triethanol amine (89%)                                                          0.2 wt%
Methyl paraben                                                                  0.2 wt%
Water                                                                           79.1 wt%

<Method of evaluating compound stability>

The cosmetics were stored at 25˚C for 7 days to determine a change in viscosity. The change in viscosity was determined by measuring the viscosity at 6 rpm for 1 min. (sample temperature 20˚C) with a Brookfield viscometer using a rotor selected from various rotors depending on the measuring range. The change in viscosity is a value expressed by the following equation:

$$\text{Change in viscosity (\%)} = [(\eta2 - \eta1)/\eta1] \times 100$$

wherein $\eta1$ is the viscosity (mPa.s) of the cosmetics just after compounding, and $\eta2$ is the viscosity (mPa.s) of the cosmetics after storage.

<Method of evaluating feel>

The feel of the cosmetics just after compounding was evaluated by one specialist in 3 ranks under the following criteria:

O: Usable without sense of incongruity.
$\Delta$: Feels slightly frictional.
$\times$: Feels frictional.

Table 2

| | Example | | | Comparative example | |
|---|---|---|---|---|---|
| | 1 | 2 | 3 | 1 | 2 |
| Zinc oxide complex powder | Synthesis example 1 | Synthesis example 2 | Synthesis example 3 | Comparative synthesis example 1 | - |
| Silicone-treated zinc oxide | - | - | - | - | MZ-507S |
| Change in viscosity | -9 | -10 | -8 | -80 or more | -80 or more |
| Feel | ○ | ○ | ○ | ○ | × |

Example 4

Using the titanium oxide complex powder obtained in Synthesis Example 4, a cosmetic base with the following composition is prepared by the following process.

<Composition of cosmetic base>
(1)   Polyether-modified silicone                                       2.5 g
        (SH3775M manufactured by Dow Corning Toray Silicone Co. , Ltd.)
(2)   Silicone Oil 10cs (KF96A 10cs, Shin-Etsu Chemical Co., Ltd)   15 g
(3)   Octamethyl cyclotetrasiloxane                                    20 g
        (SH244 manufactured by Dow Corning Toray Silicone Co., Ltd.)
(4)   Dimethyl siloxane bulk polymer                                   16 g
        (Tospearl 145A manufactured by Toshiba Silicone)
(5)   The titanium oxide complex powder in Synthesis Example 4   2 g
(6)   Glycerin                                                          2 g
(7)   Ethanol                                                          4 g
(8)   Magnesium sulfate                                               1 g
(9)   Purified water                                                  37.5 g

<Process>

The ingredients (1) to (3) are uniformly mixed with one another, then the ingredients (4) and (5) are added thereto and dispersed with a disper, and then the aqueous phase ingredients (6) to (9) are uniformly mixed with one another and added thereto, and the mixture is emulsified, followed by regulating the viscosity thereof with a homomixer to produce a cosmetic base in the form of cream.

**Claims**

1.   Metal oxide complex powder obtained by polymerizing a polymerizable and unsaturated group-containing monomer via a polymerization initiation group introduced onto the surface of at least one metal oxide selected from the group consisting of zinc oxide, titanium oxide and cerium oxide.

2.   Cosmetics comprising the metal oxide complex powder of claim 1.

3.   The cosmetics according to claim 2, wherein the metal oxide is present inside of the complex powder.

4.   The cosmetics according to claim 2 or 3, wherein the metal oxide complex powder is obtained by polymerization in the presence of a transition metal complex.

5.  The cosmetics according to claim 2 or 3, wherein the metal oxide complex powder is obtained by polymerization in the presence of a halogenated copper and an organic compound capable of coordination with copper.

6.  The cosmetics according to any of claims 2 to 5, wherein the polymerization initiation group is a halogenated alkyl group.

7.  Use of the metal oxide complex powder of claim 1 for cosmetics.

<table>
<tr><td colspan="2" align="center">**INTERNATIONAL SEARCH REPORT**</td><td>International application No.</td></tr>
<tr><td colspan="2"></td><td>PCT/JP2005/006722</td></tr>
</table>

A.  CLASSIFICATION OF SUBJECT MATTER
    Int.Cl$^7$  C09C3/10, 1/04, 1/36, A61K7/00, 7/021

According to International Patent Classification (IPC) or to both national classification and IPC

B.  FIELDS SEARCHED

Minimum documentation searched (classification system followed by classification symbols)
    Int.Cl$^7$  C09C3/10, 1/04, 1/36, A61K7/00, 7/021

Documentation searched other than minimum documentation to the extent that such documents are included in the fields searched

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2001-521972 A  (Cabot Corp.),<br>13 November, 2001 (13.11.01),<br>(Particularly, pages 19, 28)<br>& EP 1178085 A2      & US 2002-98144 A1 | 1<br>1-7 |
| Y | JP 2001-302943 A  (JSR Corp.),<br>31 October, 2001 (31.10.01),<br>(Particularly, Claims 1, 7)<br>(Family: none) | 1-7 |
| Y | JP 6-336558 A  (Kose Corp.),<br>06 December, 1994 (06.12.94),<br>(Particularly, Claim 1)<br>(Family: none) | 1-7 |

☐  Further documents are listed in the continuation of Box C.     ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 August, 2005 (29.08.05) | 13 September, 2005 (13.09.05) |

| Name and mailing address of the ISA/<br>    Japanese Patent Office | Authorized officer |
|---|---|
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11080588 A

- JP 3205249 B

**Non-patent literature cited in the description**

- *Chemical Review,* 2001, vol. 101, 2921-2990
- *Chemical Review,* 2001, vol. 101, 2935-2940

- *Chemical Reviews,* 2001, vol. 101, 2941